# EUROPEAN PATENT APPLICATION

(11) **EP 1 736 143 A1**
(43) Date of publication of application: **27.12.2006**
(21) Application number: 05720822.5
(22) Date of filing: 15.03.2005
(51) Int. Cl.: A61K 9/24, A61K 31/426, A61K 47/02, A61K 47/10, A61K 47/14, A61K 47/32, A61K 47/38, A61P 1/04, A61J 3/00

(54) **PROCESS FOR PRODUCING MEDICINE**

(30) Priority: 17.03.2004 JP 2004076321; 09.06.2004 JP 2004171549
(71) Applicant: Lintec Corporation, Tokyo 173-0001 (JP)
(72) Inventor: NOGAMI, Eiji, Saitamashi, Saitama 3360026 (JP)
(74) Representative: Benson, John Everett
(86) International application number: PCT/JP2005/004567
(87) International publication number: WO 2005/087205

(57) **Abstract**

With the object of providing a method for producing a pharmaceutical composition comprising a drug-containing layer, a first functional layer provided to one side of the drug-containing layer either directly or via an intermediate layer, and a second functional layer provided to the other side of the drug-containing layer either directly or via an intermediate layer, wherein the time during which the drug is heated is minimized and the number of steps required to produce the pharmaceutical composition is also minimized, a first intermediate 11a is prepared comprising a first drug-containing layer 3a containing a thermoplastic polymer as a base as well as a first functional layer 2a provided to one side of the first drug-containing layer 3a, with the other side of the first drug-containing layer 3a being exposed, and a second intermediate 11b is prepared comprising a second drug-containing layer 3b containing a thermoplastic polymer as a base as well as a second functional layer 2b provided to one side of the second drug-containing layer 3b, with the other side of the second drug-containing layer 3b being exposed, and the drug-containing layer 3a of the first intermediate 11a is heat fused to the drug-containing layer 3b of the second intermediate 11b.

## Description

### TECHNICAL FIELD

The present invention relates to a method for producing a pharmaceutical composition.

### BACKGROUND ART

Compliance may be diminished in the case of an orally administered pharmaceutical composition if it is rejected because the drug is bitter, astringent or otherwise unpleasant or if it causes nausea or vomiting.

Therefore, orally administered pharmaceutical compositions have been developed comprising a functional layer (a layer having a particular function) provided to both sides of a drug-containing layer either directly or via an intermediate layer. An example of this is an orally administered pharmaceutical composition comprising a water-swellable gel-forming layer provided to both sides of a drug-containing layer either directly or via an intermediate layer (International Publication WO 02/087622).

The water-swellable gel-forming layer of this orally administered pharmaceutical composition swells from the saliva inside the patient's mouth, forming a gel. Since this orally administered pharmaceutical composition changes to a form of an easily ingestible size, shape, elasticity, viscosity and the like, it can be taken easily and poses little risk of blocking the patient's trachea, so it can be given safely to elderly patients and infants. When a gel cannot form because a patient does not have enough saliva to swell the water-swellable gel-forming layer, the same effects can be achieved by giving the pharmaceutical composition together with a small amount of water or by soaking it in water before administration. Much less water is required in this case than is required for administering a tablet, capsule or other solid preparation.

Moreover, since the drug-containing layer of this orally administered pharmaceutical composition is covered on both sides with the water-swellable gel-forming layer, the flavor (for example bitterness or astringency) and smell of the drug contained in the drug-containing layer is masked and does not detract from compliance.

It is also possible to minimize the water content of the preparation by working this orally administered pharmaceutical composition into a film preparation (sheet preparation), thereby enhancing the stability of the drug (particularly in the case of easily hydrolyzed drugs) as well as making it easier to handle and reducing packaging costs.

Moreover, since in this orally administered pharmaceutical composition the drug-containing layer and water-swellable gel-forming layer are formed independently, even if the film strength of the drug-containing layer decreases when the amount of drug in the drug containing-layer is increased, the strength of the film preparation as a whole is maintained because of the film formability contributed by the water-swellable gel-forming layer. Consequently, the drug-containing layer of this orally administered pharmaceutical composition may contain a wide variety of drugs in tiny to large quantities, as well as insoluble and bulky drugs that are likely to detract from film strength.

International Publication WO 02/087622 discloses the following producing method as a first method for producing such an orally administered pharmaceutical composition. A water-swellable gel-forming layer forming liquid (with purified water for example as the solvent) containing a water-swellable gel-forming agent is painted, sprayed or the like on a plastic film, mount or other support, and then dried to form a water-swellable gel-forming layer. Next, a drug-containing layer forming liquid (with ethanol for example as the solvent) containing a drug and additives such as excipients, binders, disintegrators and the like is painted, sprayed or the like onto the water-swellable gel-forming layer, and dried to form a drug-containing layer. Next, the water-swellable gel-forming layer forming liquid is painted, sprayed or the like in the same way onto the drug-containing layer, and dried to form a water-swellable gel-forming layer.

International Publication WO 02/087622 also discloses the following producing method as a second method for producing this orally administered pharmaceutical composition. A water-swellable gel-forming layer is formed as described above onto a plastic film, mount or other support, and a drug-containing layer is then formed as described above onto the water-swellable gel-forming layer. Next, an adhesive layer forming liquid containing an adhesive agent which becomes adhesive when heated is painted, sprayed or the like onto the drug-containing layer, and dried to form an adhesive layer. An intermediate is thus produced comprising a water-swellable gel-forming layer, a drug-containing layer and an adhesive layer layered in that order on a support, and the adhesive layers of two intermediates are then heat-fused to one another.

However, because in the first producing method or second producing method described above the water-swellable gel-forming layer forming liquid or adhesive layer forming liquid which is painted or sprayed on the drug-containing layer seeps into the drug-containing layer and the water-swellable gel-forming layer below it, long-term heating is required to dry the water-swellable gel-forming layer forming liquid or adhesive layer forming liquid painted or sprayed on the drug-containing layer. As a result, in this first producing method or second producing method there is a risk that long-term heating will destabilize the drug in the drug-containing layer. Moreover, the second producing method involves more steps than the first producing method. The greater the number of steps, the greater the time, labor and costs, so it is desirable to minimize the number of steps.

### DISCLOSURE OF THE INVENTION

It is therefore an object of the present invention to provide a method for producing a pharmaceutical composition comprising a drug-containing layer, a first functional layer (such as a water-swellable gel-forming layer) provided to one side of the drug-containing layer either directly or via an intermediate layer, and a second functional layer (such as a water-swellable gel-forming layer) provided to the other side of the drug-containing layer either directly or via an intermediate layer, which is a pharmaceutical composition producing method wherein the time during which the drug is heated is made as short as possible, and the number of steps required to producing the pharmaceutical composition is minimized.

To solve these problems, the present invention provides a method for producing a pharmaceutical composition comprising a drug-containing layer, a first functional layer provided to one side of the drug-containing layer either directly or via an intermediate layer, and a second functional layer provided to the other side of the drug-containing layer either directly or via an intermediate layer, wherein the producing method comprises the following steps (a), (b) and (c):
(a) a step of preparing a first intermediate comprising a first drug-containing layer containing a thermoplastic polymer as a base and a first functional layer provided to one side of the first drug-containing layer either directly or via an intermediate layer, with the other side of the first drug-containing layer being exposed;
(b) a step of preparing a second intermediate comprising a second drug-containing layer containing a thermoplastic polymer as a base and a second functional layer provided to one side of the second drug-containing layer either directly or via an intermediate layer, with the other side of the second drug-containing layer being exposed; and
(c) a step of heat-fusing the first drug-containing layer of the first intermediate with the second drug-containing layer of the second intermediate.

In the producing method of the present invention, the first drug-containing layer of the first intermediate and the second drug-containing layer of the second intermediate are heat-fused together instead of painting or spraying and then drying a second functional layer forming liquid on a drug-containing layer or an intermediate layer formed thereon when forming a second functional layer either directly or via an intermediate layer on the other side of a drug-containing layer on one side of which a first functional layer has already been formed either directly or via an intermediate layer. The heating time required for heat-fusing the first drug-containing layer of the first intermediate with the second drug-containing layer of the second intermediate is shorter than the heating time required to dry the second functional layer forming liquid painted or sprayed on the drug-containing layer or intermediate layer formed thereon. Moreover, the heating time required to form the first drug-containing layer (drying time of first drug-containing layer forming liquid) and the heating time required to form the second drug-containing layer (drying time for second drug-containing layer forming liquid) are together shorter than the heating time required to form the one drug-containing layer of the final produced pharmaceutical composition from beginning to end. Consequently, the stability of the drug in the first drug-containing layer and second drug-containing layer can be ensured in the producing method of the present invention.

Moreover, since in the producing method of the present invention the first drug-containing layer and second drug-containing layer contain a thermoplastic polymer, there is no need to form adhesive layers on the first drug-containing layer and second drug-containing layer for purposes of heat-fusing the first intermediate to the second intermediate.

In a preferred mode of the producing method of the present invention, the pharmaceutical composition is an orally administered pharmaceutical composition, and the thermoplastic polymer is a thermoplastic edible polymer. In this mode, this thermoplastic edible polymer is preferably one or two or more thermoplastic edible polymers selected from the group consisting of polyvinylpyrrolidone, vinylpyrrolidone-vinyl acetate copolymer and polyvinyl acetate. Moreover, in this mode the first functional layer and/or second functional layer are preferably water-swellable gel-forming layers.

The present invention provides a method for producing a pharmaceutical composition comprising a drug-containing layer, a first functional layer (such as a water-swellable gel-forming layer) provided to one side of the drug-containing layer either directly or via an intermediate layer, and a second functional layer (such as a water-swellable gel-forming layer) provided to the other side of the drug-containing layer either directly or via an intermediate layer, which is a pharmaceutical composition producing method wherein the time during which the drug is heated is minimized, and the number of steps required to producing the pharmaceutical composition is also minimized.

### BRIEF DESCRIPTION OF THE DRAWINGS

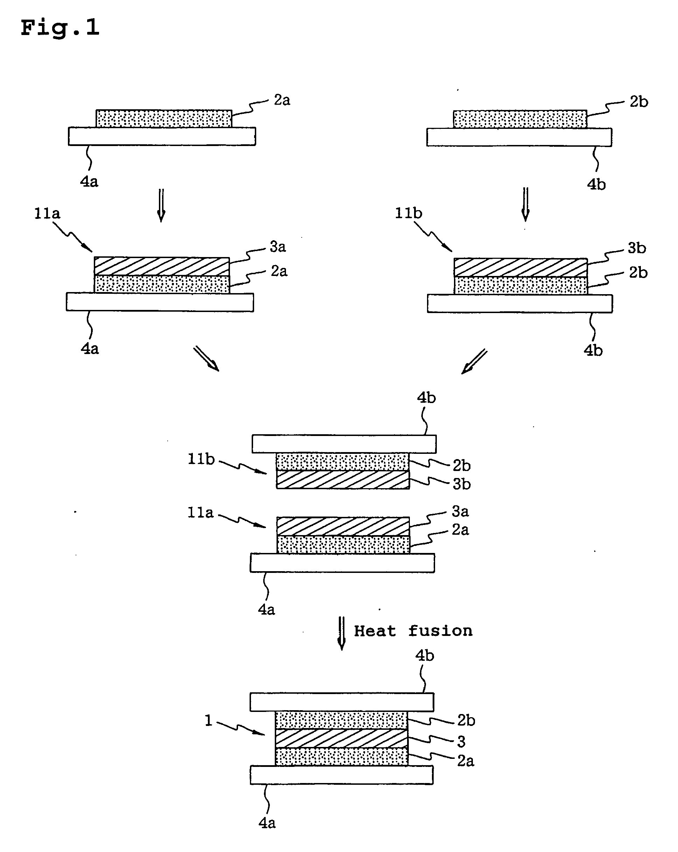

Figure 1 is a view explaining the various steps in the pharmaceutical composition producing method of the present invention.

### BEST MODE FOR CARRYING OUT THE INVENTION

The present invention is explained in detail below based on the drawings.

Figure 1 is a model view of one embodiment of the producing method of the present invention.

As shown in Figure 1, the producing method of this embodiment is a method for producing pharmaceutical composition 1 comprising drug-containing layer 3, first functional layer 2a, which is provided directly on one side of drug-containing layer 3, and second functional layer 2b, which is provided directly on the other side of drug-containing layer 3.

Pharmaceutical composition 1 is preferably a film preparation. By making it as a film preparation it is possible to minimize the water content of the preparation, thereby making the drug contained in the preparation (particularly if it is an easily hydrolysable drug) more stable than in a gelatinous preparation with a high water content. The preparation is also easier to handle, and packaging costs are reduced. The thickness of drug-containing layer 3 can be adjusted appropriately according to the drug content and the like, but when pharmaceutical composition 1 is a film preparation, drug-containing layer 3 is preferably 0.1 to 1000 µm or more preferably 10 to 200 µm thick. If the thickness of drug-containing layer 3 is under 0.1 µm it is hard to form the film precisely (that is, variation occurs in the amount of drug in drug-containing layer 3), while if the thickness is over 1000 µm the film will be stiff and harder to administer.

The type of pharmaceutical composition 1 is not particularly limited, but examples include orally administered pharmaceutical compositions and those applied to the oral mucous membrane and the like.

As shown in Figure 1, pharmaceutical composition 1 is produced by heat-fusing first intermediate 11a with second intermediate 11b.

As shown in Figure 1, first intermediate 11a comprises first functional layer 2a which is provided directly on support 4a, and first drug-containing layer 3a which is provided directly on first functional layer 2a. In first intermediate 11a, one side of first drug-containing layer 3a has first functional layer 2a provided thereon, while the other side is exposed.

As shown in Figure 1, second intermediate 11b comprises second functional layer 2b which is provided directly on support 4b, and second drug-containing layer 3b which is provided directly on second functional layer 2b. In second intermediate 11b, one side of second drug-containing layer 3b has second functional layer 3b provided thereon, while the other side is exposed.

As shown in Figure 1, when heat-fusing first intermediate 11a and second intermediate 11b the exposed surface of first drug-containing layer 3a of first intermediate 11a is brought face to face with the exposed surface of second drug-containing layer 3b of second intermediate 11b, and first drug-containing layer 3a and second drug-containing layer 3b are heat-fused with each other.

The conditions for heat fusion can be adjusted appropriately according to the type of thermoplastic polymer contained in first drug-containing layer 3a and second drug-containing layer 3b. During heat fusion, the temperature is normally 60 to 150°C or preferably 90 to 120°C, the pressure is normally 0.1 kgf/cm² or more or preferably 0.5 kgf/cm² or more, and the treatment time is 0.1 to 5 seconds or preferably 0.5 to 3 seconds.

As shown in Figure 1, first intermediate 11a can be produced by forming first functional layer 2a on support 4a, and then forming first drug-containing layer 3a on first functional layer 2a. Moreover, as shown in Figure 1, second intermediate 11b can be produced by forming second functional layer 2b on support 4b, and then forming second drug-containing layer 3b on second functional layer 2b. However, the method of producing first intermediate 11a and second intermediate 11b is not limited to this.

First functional layer 2a can be formed on support 4a by for example painting, spraying or otherwise applying a first functional layer forming liquid containing the components of the first functional layer to support 4a, and then drying it. The amount of the first functional layer forming liquid which is painted, sprayed or the like on support 4a can be adjusted as necessary. The solvent for the first functional layer forming liquid can be selected appropriately according to the type of solute, but specific examples are purified water and ethanol. The drying temperature is normally 50 to 120°C or preferably 60 to 90°C, and the drying time is normally 1 to 15 minutes or preferably 1 to 10 minutes. Second functional layer 2b can be formed in the same way on support 4b.

First drug-containing layer 3a can be formed on first functional layer 2a by for example first painting, spraying or otherwise applying a drug-containing layer forming liquid containing the components of the drug-containing layer to first functional layer 2a, and then drying it. The amount of the drug-containing layer forming liquid which is painted, sprayed or the like on first functional layer 2a can be adjusted as necessary. The solvent for the drug-containing layer forming liquid can be selected appropriately according to the type of solute, with specific examples including ethanol, ethyl acetate, toluene and purified water. The drying temperature is normally 50 to 120°C or preferably 60 to 90°C, and the drying time is normally 1 to 15 minutes or preferably 1 to 10 minutes. Second drug-containing layer 3b can be formed in the same way on second functional layer 2b.

Supports 4a and 4b are not particularly limited as to type, but examples include polyethylene terephthalate film, polypropylene film and other resin films and glassine paper, clay-coated paper, laminated paper (especially polyethylene laminated paper) and other papers which can be release-treated with a silicone release agent or the like as necessary. Supports 4a and 4b may be of the same type or of different types.

First functional layer 2a and second functional layer 2b are layers having a specific function, but the function itself is not particularly limited. First functional layer 2a and second functional layer 2b may have the same function or different functions. When pharmaceutical composition 1 is an orally administered pharmaceutical composition, first functional layer 2a and/or second functional layer 2b are preferably water-swellable gel-forming layers. A "water-swellable gel-forming layer" is a layer containing a water-swellable gel-forming agent that can swell to form a gel in the presence of moisture.

The thicknesses of first functional layer 2a and second functional layer 2b can be adjusted as necessary. When first functional layer 2a and/or second functional layer 2b are water-swellable gel-forming layers, the water-swellable gel-forming layer is preferably 10 to 1000 µm or more preferably 20 to 500 µm thick. If the water-swellable gel-forming layer is less than 10 µm thick the gel will not form properly, and the ability of the water-swellable gel-forming layer to mask the flavor and/or smell of the drug will be inadequate, while if the water-swellable gel-forming layer is over 1000 µm thick it will be difficult to ingest because it will not swell adequately to form a gel simply from the saliva inside the patient's mouth.

The water-swellable gel-forming agent contained in the water-swellable gel-forming layer is not particularly limited as to type as long as it can swell from moisture to form a gel, and it may or may not be crosslinked. Specific examples of water-swellable gel-forming agents include carboxyvinyl polymers, starch and derivatives thereof, agar, alginic acid, arabinogalactan, galactomannan, cellulose and derivatives thereof, carrageen, dextran, tragacanth, gelatin, pectin, hyaluronic acid, gellan gum, collagen, casein, xanthan gum and the like.

Of these water-swellable gel-forming agents, a crosslinked carboxyvinyl polymer is preferred, particularly crosslinked polyacrylic acid. Crosslinked carboxyvinyl polymers and crosslinked polyacrylic acid in particular do not adversely affect the film-forming capabilities of film-forming agents, and exhibit good gel strength when swollen.

Crosslinking can be accomplished with a crosslinking agent suited to the type of molecule to be crosslinked. For example, a carboxyvinyl polymer can be crosslinked with a polyvalent metal compound. Specific examples of such polyvalent metal compounds include calcium chloride, magnesium chloride, aluminum chloride, aluminum sulfate, potassium alum, iron alum chloride, ammonium alum, ferric sulfate, aluminum hydroxide, aluminum silicate, aluminum phosphate, iron citrate, magnesium oxide, calcium oxide, zinc oxide, zinc sulfate and the like.

Since when pharmaceutical composition 1 is a film preparation the water-swellable gel-forming layer needs to be made in film form, a film-forming agent is preferably included in the water-swellable gel-forming layer in order to improve the film formability of the water-swellable gel-forming layer. In this case, the content of the water-swellable gel-forming agent in the water-swellable gel-forming layer is preferably 15 to 70% by weight, while the content of the film-forming agent is preferably 30 to 85% by weight.

The film-forming agent included in the water-swellable gel-forming layer is not particularly limited as to type as long as it has film-forming ability. Specific examples of the film-forming agent include polyvinyl alcohol, polyvinylpyrrolidone, polyvinyl acetate, polyvinyl acetate phthalate, hydroxyalkyl cellulose (such as hydroxypropyl cellulose, hydroxypropyl methyl cellulose, hydroxymethyl cellulose, hydroxyethyl cellulose), alkyl cellulose (such as methyl cellulose, ethyl cellulose), carboxyalkyl cellulose (such as carboxymethyl cellulose), (meth)acrylic acid and esters thereof, xanthan gum, carrageenan, alginic acid and the like.

The film-forming agent is preferably water soluble. When the film-forming agent is water soluble, moisture penetrates the water-swellable gel-forming layer more easily, promoting swelling and gel formation of the water-swellable gel-forming layer in the mouth.

Examples of water-soluble film-forming agents including polyvinyl alcohol, hydroxyalkyl cellulose such as hydroxypropyl cellulose and hydroxypropyl methyl cellulose, methyl cellulose, polyvinylpyrrolidone, xanthan gum, carrageenan, alginic acid and the like.

A plasticizer may be included in the water-swellable gel-forming layer in order to contribute a suitable degree of flexibility to the water-swellable gel-forming layer. Examples of plasticizers include propylene glycol, polyethylene glycol, glycerin and sorbitol, glycerin triacetate, diethyl phthalate and triethyl citrate, lauric acid, sucrose, sorbitol and the like.

A masking agent may be included in the water-swellable gel-forming layer in order to mask the taste and smell of the drug. Including a masking agent in the water-swellable gel-forming layer serves to improve the effectiveness of the water-swellable gel-forming layer in masking the taste and smell of the drug, effectively preventing a decrease in drug compliance. Examples of masking agents include those that contribute acidity such as citric acid, tartaric acid and fumaric acid, sweeteners such as saccharine, glycyrrhizic acid, sucrose, fructose and mannitol, cooling agents such as menthol and peppermint oil, and natural and artificial aromatics and the like.

When polyvinyl alcohol and the like are included as film-forming agents in the water-swellable gel-forming layer, these film-forming agents may also serve as masking agents. It is desirable to use a film-forming agent having such a masking effect, and it is similarly desirable to use a water-swellable gel-forming agent having such a masking effect.

Preservatives such as methyl and propyl hydroxybenzoate and colorants such as edible lake colorants can also be included in the water-swellable gel-forming layer.

In general, mixing of these additives weakens a water-swellable gel-forming layer formed as a film, making moisture more likely to penetrate the water-swellable gel-forming layer, so that the water-swellable gel-forming layer swells and forms a gel more easily due to moisture penetrating the water-swellable gel-forming layer.

First drug-containing layer 3a and second drug-containing layer 3b contain the drug to be administered along with a thermoplastic polymer as a base.

The drug contained in first drug-containing layer 3a and second drug-containing layer 3b is a drug to be administered to a patient, with no particularly restrictions as to type. When pharmaceutical composition 1 is an orally administered pharmaceutical composition, drugs that may be contained in first drug-containing layer 3a and second drug-containing layer 3b include for example drugs that affect the central nervous system, such as amobarbital, estazolam, triazolam, nitrazepam, pentobarbital and other sleeping drugs, amitriptyline hydrochloride, imipramine hydrochloride, oxazolam, chlordiazepoxide, chlorpromazine, diazepam, sulpiride, haloperidol and other psychotropics, trihexyphenidyl, levodopa and other antiparkinsonians, aspirin, isopropylantipyrine, indomethacin, diclofenac sodium, mefenamic acid, streptokinase, streptodornase, serrapeptase, pronase and other analgesics and antiinflammatories, and ATP, vinpocetine and other central nervous metabolism enhancers; drugs that affect the respiratory system, such as carbocysteine, bromhexine hydrochloride and other expectorants, and azelastine hydrochloride, oxatomide, theophylline, terbutaline sulfate, tranilast, procaterol, ketotifen fumarate and other anti-asthmatics; drugs that affect the circulatory system, such as aminophylline, digitoxin, digoxin and other cardiac stimulants, ajmaline, disopyramide, procainamide hydrochloride, mexiletine hydrochloride and other antiarrhythmics, amyl nitrite, alprenolol hydrochloride, isosorbide dinitrate, nicorandil, oxyfedrine, dipyridamole, dilazep hydrochloride, diltiazem hydrochloride, nitroglycerine, nifedipine, verapamil hydrochloride and other anti-angina drugs, kallidinogenase and other peripheral vasodilators, atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine and other anti-hypertensives, and clofibrate, dextran sulfuric acid, nicomol, niceritrol and other anti-arteriosclerotics; blood and hematopoietic drugs, such as carbazochrome sodium sulfate, tranexamic acid and other hemostatics, ticlopidine hydrochloride, warfarin potassium and other anti-thrombosis drugs, and iron sulfate and other anemia treatment drugs; drugs that affect the digestive tract, such as azulene, aldioxa, cimetidine, ranitidine hydrochloride, famotidine, teprenone, rebamipide and other anti-ulcer drugs, domperidone, metoclopramide and other antiemetics, sennoside and other cathartics, digestive enzyme regulators, and glycyrrhizin, liver extract preparations and other drugs for treatment of liver disease; drugs that affect metabolic conditions, such as glibenclamide, chlorpropamide, tolbutamide and other anti-diabetic drugs, and allopurinol, colchicine and other gout treatment drugs; ophthalmological drugs such as acetazolamide; otorhinological drugs, such as diphenidol hydrochloride, betahistine mesylate and other antivertigo drugs; chemotherapy drugs and antibiotics, such as isoniazid, ethambutol hydrochloride, ofloxacin, erythromycin stearate, cefaclor, norfloxacin, fosfomycin calcium, minocycline hydrochloride, rifampicin and rokitamycin; anti-malignant tumor drugs, such as cyclophosphamide and tegafur; immunosuppressors such as azathioprine; hormones and endocrine treatment drugs such as luteal hormone, salivary gland hormone, thiamazole, prednisolone, betamethasone, liothyronine and levothyroxine; autacoids such as clemastine fumarate, chlorpheniramine maleate and other antihistamines; and alfacalcidol, cobamamide, tocopherol nicotinate, mecobalamin and other vitamins and the like.

The drug contained in first drug-containing layer 3a and the drug contained in second drug-containing layer 3b may be of the same or different types. Moreover, first drug-containing layer 3a and second drug-containing layer 3b may each contain one type of drug, or may contain two or more types of drugs.

The amount of drug contained in first drug-containing layer 3a and second drug-containing layer 3b may be adjusted appropriately according to the type of drug and the like. The combined amount of the drugs contained in first drug-containing layer 3a and second drug-containing layer 3b is the amount of drug contained in drug-containing layer 3 of pharmaceutical composition 1. The amount of drug contained in first drug-containing layer 3a and second drug-containing layer 3b is normally 80% by weight or less, or preferably 40% by weight or less, or more preferably 30% by weight or less of first drug-containing layer 3a and second drug-containing layer 3b. If the amount of drug exceeds 80% by weight of first drug-containing layer 3a and second drug-containing layer 3b, the film strength of the film preparation will decline. The lower limit on the amount of drug contained in first drug-containing layer 3a and second drug-containing layer 3b is normally about 0.001% by weight.

The thermoplastic polymer contained in first drug-containing layer 3a and second drug-containing layer 3b is not particularly limited as long as it is thermoplastic. When pharmaceutical composition 1 is an orally administered pharmaceutical composition, the thermoplastic polymer is a thermoplastic highly-edible polymer, and specific examples include polyvinylpyrrolidone, vinylpyrrolidone-vinyl acetate copolymer, polyvinyl acetate and the like.

The thermoplastic polymers contained in first drug-containing layer 3a and second drug-containing layer 3b may be of the same type or different types. First drug-containing layer 3a and second drug-containing layer 3b may each contain only one type of thermoplastic polymer, or may contain 2 or more types of thermoplastic polymers.

The amount of the thermoplastic polymer contained in first drug-containing layer 3a and second drug-containing layer 3b may be adjusted appropriately according to the type of thermoplastic polymer and the like, but is normally 20% by weight or more, or preferably 40% by weight or more of first drug-containing layer 3a and second drug-containing layer 3b. If the thermoplastic polymer constitutes less than 20% by weight, first drug-containing layer 3a and second drug-containing layer 3b will not form properly. The upper limit on the amount of the thermoplastic polymer contained in first drug-containing layer 3a and second drug-containing layer 3b is 100% minus the minimum content of the drug contained in first drug-containing layer 3a and second drug-containing layer 3b, and can be set appropriately according to the type of drug and the like.

In addition to the thermoplastic polymer, pharmacologically acceptable excipients, binders, disintegrators and other additives as well as masking agents, colorants and the like can be included in first drug-containing layer 3a and second drug-containing layer 3b as bases for maintaining the drug to be administered in the desired condition in the drug-containing layers.

In first intermediate 11a and second intermediate 11b, first functional layer 2a and second functional layer 2b are directly provided on support 4a and support 4b, respectively, but first functional layer 2a and second functional layer 2b can also be provided via an intermediate layer on support 4a and support 4b, respectively. Also, although in first intermediate 11a and second intermediate 11b first drug-containing layer 3a and second drug-containing layer 3b are provided directly on first functional layer 2a and second functional layer 2b, respectively, first drug-containing layer 3a and second drug-containing layer 3b can also be provided via an intermediate layer on first functional layer 2a and second functional layer 2b, respectively. In this case, a pharmaceutical composition is produced comprisig drug-containing layer 3, first functional layer 2a provided to one side of drug-containing layer 3 either directly or via an intermediate, and second functional layer 2b provided to drug-containing layer 3 either directly or via an intermediate.

There are no particular limits on the type of intermediate layer, but specific examples include drug-containing layers, functional layers and adhesive layers. There may be one type or two or more types of intermediate layer.

The adhesive contained in an adhesive layer is not particularly limited as long as it is pharmacologically acceptable, and specific examples include carboxyvinyl polymers, sodium polyacrylate and other polyacrylic acids or pharmacologically acceptable non-toxic salts thereof, acrylic acid copolymers or pharmacologically acceptable salts thereof, carboxymethylcellulose, sodium salts and other hydrophilic cellulose derivatives, pullulan, povidone, karaya gum, pectin, xanthan gum, tragacanth, alginic acid, gum arabic, acidic polysaccharides and derivatives and pharmacologically acceptable salts thereof and the like.

Pharmaceutical composition 1 may be cut into a round, oval, polygonal or other shape or may have slits cut therein.

### Examples

The present invention is explained in detail below using examples and the like, but the scope of the present invention is not limited by these examples and the like.
(1) Preparation of water-swellable gel-forming layer forming liquid (Coating Liquid A)
Coating Liquid A was prepared for purposes of forming a water-swellable gel-forming layer. The preparation procedures were as follows. 1.0 g of calcium chloride was added to 140 g of purified water, and completely dissolved by agitation for about 10 minutes. 6 g of polyacrylic acid (Junlon PW-111, Nihon Junyaku Co., Ltd.) was then gradually added to the solution with agitation, and completely dissolved by agitation for about 1 hour. 17 g of polyvinyl alcohol (Gohsenol EG-05T, Nippon Gohsei) was added gradually to the solution with agitation, and completely dissolved by agitation with heating at 70°C for about 1 hour.
Polyacrylic acid is crosslinked by calcium ions produced by ionization of calcium chloride, and the crosslinked polyacrylic acid serves as a water-swellable gel-forming agent, while the polyvinyl alcohol serves as a film-forming agent.
(2) Preparation of drug-containing layer forming liquid (Coating Liquid B)
Coating Liquid B was prepared with the composition shown in Table 1 for purposes of forming the drug-containing layer. The preparation procedures were as follows. 7 g of famotidine and 0.2 g of titanium oxide were added to ethanol or ethyl acetate, and thoroughly dispersed with a homogenizer. Next, 15 g of hydroxypropyl cellulose (hereunder "HPC", SL grade, Nippon Soda Co., Ltd.), polyvinylpyrrolidone (hereunder "PVP", Plasdone K-29/32, ISP Japan), vinyl pyrrolidone-vinyl acetate copolymer (hereunder "P(VP-VAc)", Plasdone S-630, ISP Japan) or polyvinyl acetate (hereunder "PVAc", Gohsenyl NZ-32, Nippon Gohsei) and 8 g of glycerin (Kanto Chemical Co., Inc.) were added as the solution was agitated, and completely dissolved by 1 hour of agitation. The amount of solvent was adjusted so as to obtain a solution viscosity of 2000 to 6000 Pa•s. PVP, P(VP-VAc) and PVAc are thermoplastic polymers which can be thermally fused.

**[Table 1]**

| | Recipe 1 (Example 1) | Recipe 2 (Example 2) | Recipe 3 (Example 3) | Recipe 4 (Comp.Exam ples 1, 2) |
|---|---|---|---|---|
| Famotidine | 7 g | 7 g | 7 g | 7 g |
| Titanium oxide | 0.2 g | 0.2 g | 0.2 g | 0.2 g |
| Glycerin | 8 g | 8 g | 8 g | 8 g |
| Ethanol | As necessary | As necessary | | As necessary |
| Ethyl acetate | | | As necessary | |
| HPC | | | | 15 g |
| PVP | 15 g | | | |
| P(VP-VAc) | | 15 g | | |
| PVAc | | | 15 g | |

(3) Preparation of adhesive layer forming liquid (coating liquid C)
Coating liquid C was prepared for purposes of forming an adhesive layer. The preparation procedures were as follows. 10 g of PVAc was first added to ethyl acetate, and completely dissolved by agitation for about 1 hour. PVAc is a thermoplastic polymer which can be thermally fused.

### [Examples 1, 2, 3]

Coating liquid A was thoroughly degassed and, using an applicator with the gaps aligned so as to achieve a dried coating volume of 30 g/m², spread on the non-release-treated side of a polyethylene terephthalate film (Lintec Corporation, SP-PET3801) which had been release treated on the opposite side with a silicone resin, and dried for 10 minutes at 80°C to form a water-swellable gel-forming layer.

Coating liquid B (Recipes 1, 2 and 3 in Table 1) was thoroughly degassed and, using an applicator with the gaps aligned so as to achieve a dried coating volume of 70 g/m², spread on the aforementioned water-swellable gel forming layer and dried for 10 minutes at 80°C to form a first intermediate and second intermediate comprising a water-swellable gel-forming layer and drug-containing layer layered in that order on the aforementioned polyethylene terephthalate film.

Next, the drug-containing layers of the first intermediate and second intermediate were heat fused to each other for 2 seconds under conditions of 100°C, 1 kgf/cm².

An orally administered pharmaceutical composition was thus produced comprising a water-swellable gel-forming layer, a drug-containing layer and a water-swellable gel-forming layer layered in that order on the aforementioned polyethylene terephthalate film. [Comparative Example 1]

A water-swellable gel-forming layer was formed as in Example 1 on the non-release-treated surface of a polyethylene terephthalate film (Lintec Corporation, SP-PET3801) which had been release treated on the opposite side with a silicone resin.

Coating liquid B (recipe 4 in Table 1) was thoroughly degassed, and using an applicator with the gaps aligned so as to achieved a dried coating volume of 140 g/m², spread on the aforementioned water-swellable gel-forming layer and dried for 20 minutes at 80°C to form a drug-containing layer.

Coating liquid A was thoroughly degassed and spread on the aforementioned drug-containing layer using an applicator with the gaps aligned so as to achieve a dried coating volume of 30 g/m², and dried for 15 minutes at 80°C to form a water-swellable gel-forming layer.

An orally administered pharmaceutical composition was thus produced comprising a water-swellable gel-forming layer, a drug-containing layer and a water-swellable gel-forming layer layered in that order on the aforementioned polyethylene terephthalate film. [Comparative Example 2]

A water-swellable gel-forming layer was formed as in Example 1 on the non-release-treated surface of a polyethylene terephthalate film (Lintec Corporation, SP-PET3801) which had been release treated on the opposite side with a silicone resin.

Coating liquid B (recipe 4 in Table 1) was thoroughly degassed, and using an applicator with the gaps aligned so as to achieved a dried coating volume of 70 g/m², spread on the aforementioned water-swellable gel-forming layer and dried for 10 minutes at 80°C to form a drug-containing layer.

Coating liquid C was thoroughly degassed, spread on the aforementioned drug-containing layer using an applicator with the gaps adjusted so as to achieve a dried coating volume of 10 g/m², and dried for 3 minutes at 80°C to form an adhesive layer.

A first intermediate and second intermediate were thus produced comprising a water-swellable gel-forming layer, drug-containing layer and adhesive layer layered in that order on the aforementioned polyethylene terephthalate film.

The adhesive layers of this first intermediate and second intermediate were then heat fused with each other for 2 seconds under conditions of 100°C, 1 kgf/cm².

An orally administered pharmaceutical composition was thus produced comprising a water-swellable gel-forming layer, a drug-containing layer, an adhesive layer, a drug-containing layer and a water-swellable gel-forming layer layered in that order on the aforementioned polyethylene terephthalate film.

The heating times (seconds), total numbers of steps and numbers of steps during which the drug-containing layers are heated are shown in Table 2 for Examples 1 to 3 and Comparative Examples 1 and 2.

**[Table 2]**

| | Drug heating time (secs) | Total steps | Steps during which drug-containing layer is heated |
|---|---|---|---|
| Comp. Ex. 1 | 2100 | 3 | 2 |
| Comp. Ex. 1 | 1082 | 7 | 3 |
| Example 1 | 902 | 5 | 2 |
| Example 2 | 902 | 5 | 2 |
| Example 3 | 902 | 5 | 2 |

Of the total number of steps, Comparative Example 1 (3 steps) includes 2 steps for forming the water-swellable gel-forming layer and 1 step for forming the drug-containing layer, while Comparative Example 2 (7 steps) includes 3 steps for producing the first intermediate (water-swellable gel-forming layer forming step, drug-containing layer forming step and adhesive layer forming step), 3 steps for producing the second intermediate (same steps as for first intermediate) and 1 step for heat fusing the first and second intermediates, and Examples 1 to 3 (5 steps) each include 2 steps for producing the first intermediate (water-swellable gel-forming layer forming step, drug-containing layer forming step), 2 steps for forming the second intermediate (same steps as for first intermediate), and 1 step for heat fusing the first and second intermediates.

Of the number of steps during which the drug-containing layer is heated, Comparative Example 1 (2 steps) includes a drug-containing layer forming step (1 step) and a step of forming a water-swellable gel-forming layer on the drug-containing layer (1 step), while Comparative Example 2 (3 steps) includes a drug-containing layer forming step (1 step), a step of forming an adhesive layer on the drug-containing layer (1 step) and a step of heat fusing the first and second intermediates (1 step), and Examples 1 to 3 include a drug-containing layer forming step (1 step) and a step of heat fusing the first and second intermediates (1 step), but the time during which the drug-containing layer is heated is shorter in Examples 1 to 3 than in Comparative Examples 1 and 2.

### INDUSTRIAL APPLICABILITY

The present invention provides a method of producing a pharmaceutical composition comprising a drug-containing layer, a first functional layer (such as a water-swellable gel-forming layer) provided to one side of the drug-containing layer either directly or via an intermediate, and a second functional layer (such as a water-swellable gel-forming layer) provided to the other side of the drug-containing layer either directly or via an intermediate, wherein the time during which the drug is heated is minimized, and the number of steps required to produce the pharmaceutical composition is also minimized.

## Claims

1. A method for producing a pharmaceutical composition comprising a drug-containing layer, a first functional layer provided to one side of the drug-containing layer either directly or via an intermediate layer, and a second functional layer provided to the other side of the drug-containing layer either directly or via an intermediate layer, wherein the producing method comprises the following steps (a), (b) and (c):
(a) a step of preparing a first intermediate comprising a first drug-containing layer containing a thermoplastic polymer as a base and a first functional layer provided to one side of the first drug-containing layer either directly or via an intermediate layer, with the other side of the first drug-containing layer being exposed;
(b) a step of preparing a second intermediate comprising a second drug-containing layer containing a thermoplastic polymer as a base and a second functional layer provided to one side of the second drug-containing layer either directly or via an intermediate layer, with the other side of the second drug-containing layer being exposed; and
(c) a step of heat-fusing the first drug-containing layer of the first intermediate with the second drug-containing layer of the second intermediate.

2. The producing method according to Claim 1, wherein the pharmaceutical composition is an orally administered pharmaceutical composition, and the thermoplastic polymer is a thermoplastic edible polymer.

3. The producing method according to Claim 2, wherein the thermoplastic edible polymer is one or two or more thermoplastic edible polymers selected from the group consisting of polyvinylpyrrolidone, vinylpyrrolidone-vinyl acetate copolymer and polyvinyl acetate.

4. The producing method according to Claim 2 or 3, wherein the first functional layer and/or second functional layer are water-swellable gel-forming layers.
